Europäisches Patentamt

European Patent Office

Office européen des brevets

(19)

(11) Publication number: **0 194 736**

A1

# EUROPEAN PATENT APPLICATION

(21) Application number: 86300280.4

(22) Date of filing: 16.01.86

(51) Int. Cl.⁴: **C 12 N 9/64**
C 12 N 5/00, A 61 K 37/54
//A61K37/465

(30) Priority: 12.03.85 JP 47349/85

(43) Date of publication of application:
17.09.86 Bulletin 86/38

(84) Designated Contracting States:
DE GB

(71) Applicant: KOCHI MEDICAL SCHOOL
Kohasu Okoo-Cho
Nankoku City Kochi Prefecture(JP)

(72) Inventor: Niiya, Kenji
587-75-A-402, Kamohara Okoo-Cho
Nankoku City Kochi Pref.(JP)

(72) Inventor: Kubonishi, Ichiro
642-8, Mama
Kochi City Kochi Pref.(JP)

(72) Inventor: Miyoshi, Isao
282-20, Nakamama
Kochi City Kochi Pref.(JP)

(74) Representative: Woods, Geoffrey Corlett et al,
J.A. KEMP & CO. 14 South Square Gray's Inn
London WC1R 5EU(GB)

(54) Plasminogen activator production.

(57) Plasminogen activator, useful as a thrombus dissolving agent, is produced by culturing cells of the cell line RC-K8 (ECACC 86010801) and obtaining the plasminogen activator thus-produced from the supernatant of the culture.

EP 0 194 736 A1

Croydon Printing Company Ltd.

0194736

## PLASMINOGEN ACTIVATOR PRODUCTION

This invention relates to plasminogen activator produced from a cell strain of malignant lymphoid tumor in a medical field.

In recent years, as the plasminogen activator, urokinase which exists in urine, and tissue plasminogen activator produced from vascular endothelial cells or cell strains of malignant melanoma have been known. This plasminogen activator is an enzyme which acts on plasminogen to change the latter into plasmin having action of fibrinolysis. Recently, urokinase produced from urine and from cultured renal cells are widely used as a dissolving agent of thrombus.

However, it becomes gradually difficult to collect fresh urine with its enlarged demand, and also production of urokinase which uses the cultured renal cells has a limit in supply of cells which produce urokinase. Therefore, these methods have many problems in the mass production of urokinase.

Thrombotic deseases tend to increase in severe diseases such as myocardial infarction, cerebral apoplexy etc., so that useful development of treatments for dissolving thrombus is waited. Accordingly, it is expected that frequency in use of plasminogen activator having the action of fibrinolysis will increase in future.

In order to resolve these problems, this invention provides plasminogen activator produced from a cell strain which is designated as RC-K8 and established by a malignant lymphoid tumor cell.

The above RC-K8 is a culture cell line which increases semipermanently, and it is able to produce infinitely urokinase. Therefore, according to this invention, it is possible to produce urokinase in large quantities as well as to provide plasminogen activator given in a stable price.

RC-K8 is a cell strain which was established from ascites cells of a patient with malignant lymphoid tumor. This RC-K8 cell strain is positive for human B-cell antigen (B1) and have properties of immature B lymphocytes. As this RC-K8 cell strain secretes a large quantity of plasminogen activator, plasminogen activator is obtainable from the supernatant of the culture solution by the simple technique of purification.

The following examples illustrate the process for producing plasminogen activator of this invention.

Preparation of RC-K8 cells

30 mℓ of ascites was obtained by paracentesis from a patient with malignant lymphoid tumor and the cells were collected by centrifugation. Cultures were initiated at $5 \times 10^6$ cells/mℓ in 35 mm Petri dishes (3 mℓ per dish) with RPMI 1640 medium (made by Flow Lab.) supplemented with 15% fetal calf serum and 15% human cord serum. Cell strains obtained after 3 months were designated RC-K8.

RC-K8 cells were adapted to grow in medium consisting of RPMI 1640 supplemented with 15% fetal calf serum. When the cells were seeded in flasks at $4 \times 10^5$ cells/m$\ell$, the density of cells reached the saturation density of $1 \times 10^6$ cells/m$\ell$ in 3 to 4 days with a doubling time of 48 to 60 hours.

Karyotypic analysis of RC-K8 revealed a modal chromosome number of 46 in 30 (79%) of 38 metaphases examined and several structural abnormalities including 14q+. Routine histochemical stainings showed that RC-K8 cells were positive for acid phosphatase, periodic acid-Schiff (PAS), and $\alpha$-naphthyl butyrate esterase which was inhibited by NaF. Myeloid cell specific stainings such as peroxidase, Sudan black B, naphthol AS-D chloroacetate esterase and alkaline phosphatase were all negative. The cells were negative for phagocytosis, lysozyme, and $\alpha_1$-antitrypsin. The peroxidase anti-peroxidase staining method demonstrated that RC-K8 was positive for $\alpha$ subunit of S-100 protein. Immunologic studies showed that RC-K8 had receptors for complement but not for sheep erythrocytes or Fc portion of immunoglobin. By membrane immunofluorescence assay, RC-K8 cells were shown to be positive for human B-cell antigen (B1) and Ia antigen (OKIa1), but negative for surface immunoglobulin and human T-cell antigens (Leu-1, Leu-2a, and Leu-3a), common acute lymphoblastic leukemia antigen (J5), and monocyte antigen (Mo2). Neither

- 4 -

0194736

cytoplasmic immunoglobulin nor Epstein-Barr virus nuclear antigen (EBNA) was detected.

These results showed that RC-K8 possesses a unique phenotype for both monocytes/macrophages ($\alpha$ subunit of S-100 protein) and B-cells (B1). Lack of detectable surface and cytoplasmic immunoglobulins as markers for B-cells or lysozyme and $\alpha$-antitrypsin as markers for histiocytes may reflect the poorly differentiated nature of the cells.

Preparation of plasminogen activator

$1\times10^6$ cells/m$\ell$ of RC-K8 cells were floated on Petri dishes having a diameter of 3 cm with RPMI 1640 medium containing 10% fetal calf serum, and cultured in $CO_2$ incubator under the atmosphere of 95% of air and 5% of $CO_2$ at 37°C for 3 days. Further, the cultured cells were gathered by centrifugation and washed with a culture solution three times and cultured in serum-free solution of RPMI medium for 24 hours. Plasminogen activating activity equivalent to 15-20 IU of Urokinase was found in the supernatants of the culture solution.

This plasminogen activator will be a type of urokinase because the activity was fully lost by reacting with anti-urokinase-anti-serum. Plasminogen activator produced from RC-K8 cell strain represents the activity of plasminogen activator having two kinds of molecular sizes (58,000 and 53,000) with zymography by the fibrin-plate method which contains plasminogen after the gel electrophoresis of sodium dodecyl sulfate (SDS)-polyacrylamide. The obtained molecular size partly differed from the molecular size of standard urokinase (53,000 only).

Then, the obtained RC-K8 cell strain was cultured in serum-free medium containing aprotinine which was reversible inhibitor of plasmine. The cultured supernatant newly showed the activity of plasminogen activator having a molecular weight of 100,000. After the cultured solution containing aprotinine was reacted with diisopropyl fluorophosphate, the plasminogen activator activity was proved on zymography. This fact shows the possibility of the existence of proenzyme of plasminogen activator. Parallel to this test, T-cell acute lymphocytic leukemia cell strain (TALL-1), acute promyelocytic leukemia cell strain (PL-21) and chronic myelocytic leukemia line (KCL-22) were tested for plasminogen activator. As it could be assumed that the inhibitor against plasminogen activator produced simultaneously in the cultured solutions, plasminogen activator activity was determined by SDS treatment (0.3%) in order to eliminate the inhibitor. However, the cell strains except the RC-K8 cell strain little showed the activity.

Accordingly, the above fact shows that the RC-K8 cell strain can also produce plasminogen activator of urokinase type having a high activity against hematopoietic cells under certain conditions in addition to renal cells or vascular endothelial cells as urokinase producing cells.

The effect of this invention is as follows.

1. According to this invention, the RC-K8 cell strain secretes a large amount of plasminogen activator, so that the thrombus dissolving agent which gives a stable price can be provided.

2. Retinoic acid, porbol ester, malignant virus and a certain hormone are considered as materials able to increase the production of plasminogen activator of cultured cells. In future, the production of plasminogen activator can be increased by acting on these materials with the RC-K8 cell strains.

3. It is known that plasminogen activator has not only fibrinolysis action but also many important actions of kinin production, reparation of tissues, wandering of cells, proliferation and transfer of tumor, transformation and enhancement of cells by virus. Therefore, this RC-K8 cell strain will be effective in such researches.

Samples of the RC-K8 cells were deposited at the European Collection of Animal Cell Cultures (ECACC), PHLS CAMR, Porton Down, Salisbury, Wilts SP4 OJG, GB on 8 January 1986 under deposition no. ECACC 86010801.

## CLAIMS

1. A process for producing plasminogen activator which process comprises culturing cells of the cell line RC-K8 (ECACC 86010801) and obtaining the plasminogen activator thus produced from the supernatant of the culture.

2. A process for producing plasminogen activator proenzyme, which process comprises culturing cells of the cell line RC-K8 (ECACC 86010801) in the presence of aprotinin and obtaining the proenzyme from the culture.

3. The RC-K8 (ECACC 86010801) cell line.

4. A culture of cells of the cell line RC-K8 (ECACC 86010801) in a nutrient culture medium therefor, said culture being substantially free of other microorganisms.

5. A culture according to claim 4 in which the nutrient culture medium is a synthetic culture medium containing a source of assimilable carbon, a source of assimilable nitrogen and, if desired, mineral salts.

6. A pharmaceutical composition comprising plasminogen activator produced by a process as claimed in claim 1 and a pharmaceutically acceptable carrier or diluent.

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| X | JAPANESE JOURNAL OF CANCER RESEARCH (GANN), vol. 76, no. 1, January 1985, pages 12-15; I. KUBONISHI et al.: "Establishment of a new human lymphoma line that secretes plasminogen activator" * Page 12, column 1, abstract and paragraph 1 and column 2, paragraph 1; page 13, column 2, last paragraph; page 14, column 1 * | 1,3-6 | C 12 N 9/64<br>C 12 N 5/00<br>A 61 K 37/54 //<br>A 61 K 37/465 |
| Y | Idem * Page 13, column 2, last paragraph * | 2 | |
| Y | EP-A-0 112 122 (SOUTH AFRICAN INVENTIONS) * Claim 1; page 29, lines 3-6 * | 2 | **TECHNICAL FIELDS SEARCHED (Int. Cl.4)** |
| A | CHEMICAL ABSTRACTS, vol. 97, no. 1, 5th July 1982, page 407, no. 4069e, Columbus, Ohio, US; L. SKRIVER et al.: "Plasminogen activator released as inactive proenzyme from murine cells transformed by sarcoma virus", & EUR. J. BIOCHEM. 1982, 124(2), 409-14 * Abstract * | 2 | C 12 N<br>A 61 K |

The present search report has been drawn up for all claims

| Place of search | Date of completion of the search | Examiner |
|---|---|---|
| THE HAGUE | 27-05-1986 | CUENDET P.A. |